# EUROPÄISCHE PATENTSCHRIFT

(11) **EP 0 901 792 B1**
(45) Veröffentlichungstag und Bekanntmachung des Hinweises auf die Patenterteilung: **17.11.2004**
(21) Anmeldenummer: 98108214.2
(22) Anmeldetag: 06.05.1998
(51) Int. Cl.: A61K 47/42, A61K 9/26, A61K 9/52, A61K 9/20

(54) **Collagen-Schwamm zur oralen Verabreichung zur Appetithemmung**
Sponge of collagen for oral use as appetite suppressant
Eponge de collagène pour l'administration pérorale pour couper l'appétit

(30) Priorität: 05.09.1997 DE 19739031
(43) Veröffentlichungstag der Anmeldung: 17.03.1999
(73) Patentinhaber: Dr. Suwelack Skin & Health Care AG, 48727 Billerbeck (DE)
(72) Erfinder: Suwelack, Wolfgang, 48727 Billerbeck (DE); Tewes-Schwarzer, Petra, 48308 Senden (DE); GRONING, Rüdiger, Prof. Dr., 48159 Münster (DE)
(74) Vertreter: Gille Hrabal Struck Neidlein Prop Roos

(56) Entgegenhaltungen:
- EP-A- 0 224 453
- EP-A- 0 326 151
- EP-A- 0 471 217
- EP-A- 0 518 697
- WO-A-85/04413
- WO-A-88/04923
- WO-A-95/15773
- AT-T- 71 305
- DE-A- 3 124 981
- DE-A- 4 201 179
- FR-M- 7 911
- US-A- 3 435 110
- US-A- 4 401 682
- US-A- 4 601 896
- US-A- 4 774 091
- US-A- 5 206 028
- US-A- 5 219 576

## Beschreibung

Die vorliegende Erfindung betrifft die Verwendung eines komprimierten Collagen-Schwammes zur oralen Verabreichung zur Appetithemmung. Der Collagen-Schwamm wird wenigstens zum Teil über den Verdauungstrakt abgebaut und / oder abgeführt und weist nach Expansion im Magen eine schwammartige Struktur auf.

Aus der Literatur sind zahlreiche sogenannte pharmazeutische Appetitzügler bekannt, die den Körper auf biochemischen Wege eine Abneigung zur Nahrungsaufnahme (Appetitlosigkeit) suggerieren, wie beispielsweise Amphetamin. Auch eine Reihe chemisch verwandter Verbindungen werden als Appetitzügler verwendet. Sie zählen zu den indirekt wirkenden Sympathomimetika und wirken deshalb auch indirekt glykogeno- und lipolytisch. Nicht alle Patienten sprechen auf Appetitzügler an und selbst im Falle anfänglicher Wirksamkeit lässt diese bald nach. Da die Mittel außerdem meist Nebenwirkungen erzeugen, pulmonale Hypertonie im Falle des Aminorex, psychomotorische Erregung und Gewöhnung bis zur Sucht, kommen sie nur bei speziellen Indikationen, beispielsweise zur Einleitung einer Diätbehandlung in Frage.

Neben diesen pharmazeutisch wirkenden Appetithemmern sind auch zahlreiche Versuche unternommen worden, eine entsprechende Appetithemmung durch komprimierte, nicht toxische Träger zu erreichen, die wenigstens zum Teil über den Verdauungstrakt abgebaut oder abgeführt werden, wobei derartige Träger im Magen expandiert werden und dem Magen dort ein Völlegefühl und ein entsprechendes Sattsein suggerieren.

Aus der EP-A-0 317 079 ist ein Mittel bekannt, bei dem die Inhaltsstoffe praktisch ohne Verbund, jedoch in gepresster Form in Kapseln eingebracht oder zu Tabletten verpresst werden. Nach dem Einnehmen löst sich die Kapsel bzw. die gepresste Tablette im Magen auf, die Inhaltsstoffe bilden eine gequollene Masse, die im Magen befindliche Flüssigkeit bindet und wird über den Verdauungstrakt abgebaut bzw. wieder ausgeschieden. Da das Mittel keinerlei Brennwert hat, dem Körper jedoch ein Sättigungsgefühl aufgrund der im Magen befindlichen Quellmasse vorgetäuscht wird, wird die Nahrungsaufnahme ohne große Überwindung zum Zweck der Gewichtsreduktion eingestellt bzw. vermindert werden. Da die Quellmasse im wesentlichen ungebunden ist, wird sie relativ schnell aus dem Magen in den weiteren Verdauungstrakt abgeleitet, weshalb das dadurch hervorgerufenen Sättigungsgefühl nur vergleichsweise kurze Zeit anhält.

Aus der EP-A-0 043 317 ist ein hydrophiler Stoff auf Polyurethan-Polyol-Basis bekannt, der die Eigenschaft hat, Wasser zu binden und auch zum therapeutischen und kosmetischen Zweck vorgesehen ist. Als Mittel zur Gewichtsreduktion ist dieser Stoff jedoch auch aus den vorerwähnten Gründen ungeeignet.

Weiterhin sind aus der US-A-4 401 682 sowie der WO-90/01 879 Mittel auf Basis von Cellulosefasern bekannt, denen weitere Ingredienzien zugegeben sind. Diese Cellulosefasern nebst Zusatzstoffen werden zu einem feinen Puder zerkleinert und mit Gelatine vermengt, so dass sich bei Einnahme dieser Mittel ebenfalls eine gallertartige Masse mit undefinierter Raumstruktur im Magen bildet, die vergleichsweise schnell abbaubar ist. Dies führt dazu, dass vergleichsweise große Mengen des Mittels in zeitlich kurzen Abständen eingenommen werden müssen, um das gewünschte Sättigungsgefühl zu erzeugen. Dies ist sowohl aus Kostengründen als auch wegen der mit erhöhtem Massendurchsatz auch steigenden Gefahr von Nebenwirkungen ungünstig.

Das US-Patent 4 735 214 betrifft ein Mittel zur oralen Einnahme mit einem in Wasser lösbaren und den Inhalt freigebenden Behältnis, nämlich Gelatine, das mit einem beim Freisetzen volumenvergrössernden, nicht toxischen und brennstoffarmen Stoff, hier hydrophilem geschlossenzelligen Polyurethan-Schaum, gefüllt ist, wobei der Stoff durch einen Körper mit schwammartiger Struktur gebildet ist, der in komprimierter Form im Behältnis angeordnet und durch das Behältnis in seiner komprimierten Form gehalten wird. Dieses Mittel wird über ein flexibles Band in den Magen eingebracht und nach der Durchführung eines Abstrichs zurückgeführt und dient als Kapsel zur Diagnose des Magen-Darmtraktes, beispielsweise zur Ermittlung von Magenkrebs.

Ein weiteres oral verabreichbares Mittel, welches ebenfalls ein komprimiertes Polyurethan mit einer Gelatinekapsel als Behälter enthält, ist Gegenstand des US-Patents 3 688 763. Diese Kapsel weist weiterhin allerdings noch eine äußere Beschichtung aus Celluloseacetatphthalat auf, so dass sich diese Kapsel nicht bereits schon im Magen, sondern erst im Dickdarm unter Freisetzung des Polyurethan-Schwamms auflöst.

Die europäische Patentanmeldung 0 202 159 beschreibt eine Vorrichtung aus wenigstens einem im Magen lösbaren Polymer und wenigsten einem nicht löslichen Polymer, wobei das nicht lösbare Polymer u.a. aus nicht wasserlöslichen Cellulosen ausgewählt ist.

Die europäische Patentanmeldung 0 344 939 betrifft gemäss Figur 1 ein im Magen verweilendes System mit Retard-Wirkung, wobei der Kern aus einem mikroporösen Polymer, welches durch Auflösung, Hydrolyse oder enzymatischen Abbau Festigkeit verliert. Dies sind insbesondere spezielle modifizierte Cellulosen. Die Arme dieses Systems selbst können u.a. aus Celluloseestern bestehen.

Das US-Patent 4 434 153 betrifft ein Retard-Präparat, in dem der Wirkstoff in ein hydrophiles Polymer, beispielsweise ein Hydrogel pflanzlichen oder tierischen Ursprungs oder auch synthetischen Ursprungs, eingebettet ist.

Patent Abstract of Japan C-742, July 18, 1990, Vol. 14, No. 334, eine Zusammenfassung der JP 2-121919 A, betrifft eine Tablette, die durch Mischen von 10 bis 50 Gew.-% Gelatine mit 5 bis 20 Gew.-% Glycerin, 5 bis 40 Gew.-% Polyacrylsäure sowie 0,03 bis 60 Gew.-% eines Pharmazeutikums durch Kompression in an sich bekannter Weise, gefolgt von einer kurzzeitigen Wärmebehandlung, hergestellt worden ist. Die so erstelle Tablette weist gute Retardeigenschaften im Verdauungstrakt auf. Dieser Stand der Technik gibt allerdings keine Hinweise einen Träger aus Collagen mit einer Schwammstruktur einzusetzen.

Die EP-A 2 35 363 betrifft eine Tablette mit Retardwirkung, die einen speziellen Wirkstoff zusammen mit denaturiertem Eialbumin enthält und vorzugsweise weiterhin ein Beschichtungsmittel aufweist und erhalten wird durch Trockenmischung der beiden vorgenannten Komponenten, Befeuchtung und Trocknung und Tablettenpressung, gefolgt von einer Wärmebehandlung auf wenigstens 60 °C. Hinweise zu einem Träger aus Collagen mit Schwammstruktur liefert diese Druckschrift nicht.

Die DE-C 32 02 255 betrifft ein Verfahren zur Herstellung eines Retardpräparats, welches dadurch erhalten wird, dass man ein System mit einem oder mehreren Polypeptiden, einen oder mehreren Proteinen und einer oder mehrerer physiologisch aktiven Substanzen zunächst in gefrorenem Zustand vermahlt und mechanisch mischt und in einem Folgeschritt den Formkörper zu einem Pressling umsetzt. Vorzugsweise findet im Anschluss daran eine Behandlung mit elektromagnetischer Strahlung statt. Unter Polypeptid im Sinne dieser Druckschrift versteht man spezielle Polypeptide gemäss Spalte 3, Zeilen 20-31 und unter Protein wiederum spezielle Strukturen wie Spalte 3, Zeilen 32 - 45 zu entnehmen ist. Weiterhin scheint bei dieser Patentschrift wesentlich zu sein, dass durch die Druckerwärmung von den Polypeptiden und Proteinen ein scheinbarer Film gebildet wird und auch die nachfolgende Bestrahlung mit elektromagnetischen Wellen hat offenbar zum Ziel, eine Denaturierung der Oberfläche des Presslings zu erreichen, was gemäss der vorliegenden Erfindung nicht notwendig ist.

Die EP-A-0 471 217 (und teilweise entsprechend die prioritätsbegründende DE-A 40 25 912) betrifft ein Mittel zur oralen Einnahme mit einem im Magen lösbaren und den Inhalt freigebenden Behältnis, das mit einem beim Freisetzen volumenvergrössernden, nicht toxischen und brennstoffarmen Stoff gefüllt ist, der innerhalb des Verdauungstraktes abbaubar oder über diesen abführbar ist, wobei der Stoff ein Schwamm ist, der in komprimierter Form im Behältnis angeordnet ist und durch das Behältnis in dieser komprimierten Form gehalten wird. Dieser besagte brennstoffarme Stoff ist vorzugsweise ein Cellulose-Schwamm oder ein Polyurethan-Schaumstoff. Dieses vorgenannte Verfahren ist allerdings in der Praxis wenig ausführbar, da der Einsatz eines Polyurethan-Schaumstoffs als komprimierter, expandierbarer Stoff sowohl in der Bundesrepublik Deutschland als auch in weiteren ausländischen Staaten nach der Zusatzstoff-Rahmen-Richtlinie 89/107/EWG bzw. der Richtlinie über sonstige Zusatzstoffe in der EG nicht zugelassen ist. Darüber hinaus scheint auch die spezielle erstmals in der Nachanmeldung angesprochene Schwamm- oder Aveolar-Cellulose wenigstens nach den lebensmittelrichtlichen Bestimmungen nicht zugelassen, da dort ausschliesslich mikrokristalline Cellulose und Pulvercellulose, Methylcellulose, Hydroxypropylcellulose, Hydroxypropylmethylcellulose, Methyethylcellulose und Natriumcarboxymethylcellulose zugelassen sind. Auch ist das in der vorgenannten Patentanmeldung beschriebene Mittel insofern technisch aufwendig gestaltet, als es als zwingenden Bestandteil stets ein im Magen lösbares und den Inhalt freigebendes Behältnis, also konkret eine Hartgelantinekapsel voraussetzt.

Der vorliegenden Erfindung liegt die Aufgabe zugrunde, die Verwendung eines Mittels zur peroralen Verabreichung mit wenigstens einem komprimierten, nicht toxischen Träger, der wenigstens zum Teil über den Verdauungstrakt abgebaut oder abgeführt wird, wobei der Träger nach der Expansion im Magen eine schwammartige Struktur aufweist, zur Appetithemmung bereitzustellen, der gegenüber den vorgenannten einfachen Quellmassen oder Quellmassen in Schwammform technisch einfacher hergestellt wird, im Magen eine ausreichend lange Verweildauer aufweist und zu wenigstens einem Teil über den Verdauungstrakt abgebaut wird und insbesondere lebensmittelrechtlich zugelassen ist.

Diese Aufgabe wird durch die Bereitstellung der Verwendung eines komprimierten Collagen-Schwammes zur oralen Verabreichung zur Appetithemmung gelöst.

Die vorliegende Erfindung betrifft somit die Verwendung eines komprimierten Collagen-Schwammes zur oralen Verabreichung zur Appetithemmung. Der Collagenschwamm wird wenigstens zum Teil über den Verdauungstrakt abgebaut oder abgeführt und weist nach Expansion im Magen eine schwammartige Struktur auf.

Unter den erfindungsgemäß eingesetzten Collagenstrukturen versteht man im wesentlichen die sogenannten Skleroproteine, die auch unter der Bezeichnung Faserproteine, Gerüstproteine oder Strukturproteine bekannt sind und eine Gruppe von wasserunlöslichen, faserförmig aufgebauten, tierischen Proteinen mit reiner Gerüst- und Stützfunktion darstellen. Das Collagen wird aus Stütz- und Bindegeweben, Haut, Knochen und Knorpeln gewonnen.

In einer weiteren bevorzugten Ausführungsform enthält der Collagenschwamm die Aminosäuren Glycin und Hydroxyprolin, vorzugsweise mit der Tripeptidsequenz GlyXy, wobei X für eine beliebige Aminosäure und anstelle von y häufig Hydroxyprolin steht.

Nach einer weiteren bevorzugten Ausführungsform stammt der Collagenschwamm aus dem Stamm Porifera, insbesondere der Klasse Demospongiae. Hierbei handelt es sich um die zoologische Bezeichnung der umgangssprachlich als Schwamm bezeichneten Meerestiergruppe. Diese Meeresbewohner weisen eine ohne Symmetrie, jedoch polar organisierte klumpen-, krusten-, trichter- bis schüssel- aber auch pilz- und geweihförmige Gestalt auf, die durch ein Skelett aus Collagen-(Spongin) Fasern erzeugt wird, in das Skleren aus Calcit oder Kieselsäure eingelagert sind. Die Schwämme weisen meist drei Schichten auf, von denen die grösste mittlere Schicht, das Mesohyl aus einer gallertartigen Grundsubstanz mit Collagenfasern besteht. Wir verweisen beispielsweise auf das Lexikon der Biologie, Band 7, Freiburg 1986, Stichwort Schwämme, sowie ebenda Band 8, Stichworte Spongia, Spongin.

Der Stamm Porifera gliedert sich in die Klassen Calcarea, d.h. Schwämmen mit Calciteinlagerungen, Hexactinelliida, also solche mit speziellen Kieselsäureeinlagerungen sowie Desmosongiae, worunter solche mit einem Faser oder Kieselsäuregerüst fallen. Zur Gruppe der insbesondere geeigneten Klasse *Demospongiae* stammt Hierunter fallen insbesondere die Hornkieselschwämme (Cornacu-spongia), die Süsswasserschwämme und der Badeschwamm (Spongia officialis) mit den Unterarten Levantinerschwamm (Spongia officialis mollissima), Zimmoka-schwamm (Spongia officialis cimmoca), Elefantenohr (Spongis officialis lamella) sowie der Grosslöcherige Pferdeschwamm (Hippospongia Communis).

Die aus dem Wasser gewonnenen Schwämme werden in an sich bekannter Weise, beispielsweise durch sauren Aufschluss von den mineralischen Bestandteilen befreit, um hieraus den Collagenträger als wesentlichen Bestandteil des erfindungsgemäßen Mittels isolieren zu können.

Nach einer weiteren bevorzugten Ausführungsform besteht der Collagenschwamm aus Collagen, dass sich aus natürlichen tierischen Materialien ableitet. Die Herstellung dieser bevorzugt eingesetzten Collagenschwämme ist an sich bekannt, beispielsweise aus der deutschen Offenlegungsschrift 18 11 290, der deutschen Offenlegungsschrift 26 25 289, der deutschen Patentschrift 27 34 503 und insbesondere aus der deutschen Offenlegungsschrift 32 03 957 der Anmelderin.

Nach einer weiteren bevorzugten Ausführungsform weist der erfindungsgemäß verwendete Collagenschwamm vor der Komprimierung eine Dichte von 0,005 g/cm³ bis 1,0 g/cm³, vorzugsweise 0,01 bis 0,1 g/cm³ auf. Die besagte Dichte wird nach DIN 53420 gemessen.

Nach einer weiteren bevorzugten Ausführungsform ist der Collagenschwamm nicht verkapselt, liegt also als Pressling vor. In diesem Zusammenhang verweisen wir beispielhaft auf die Monographie Arzneimittelformenlehre von Frau Schöffling-Krause, Stuttgart 1987, S. 131 - 154 und die dort beschrieben Herstellmethoden und Maschinen sowie auf das Kapitel Tabletten im Buch Rudolf Voigt, Pharmazeutische Technologie für Studium und Beruf, Verlag Ullstein Mosby, Berlin 1993 S. 205 ff. und die dort beschriebenen Herstellmethoden und Maschinen hin. Die Materialzufuhr zu den Tablettenpressen wird materialgerecht modifiziert.

Nach einer weiteren bevorzugten Ausführungsform weist der Collagenschwamm im erfindungsgemäßen Mittel die Form einer Tablette auf. Wiederum verweisen wir beispielhaft auf die Monographie von Schöffling-Krause. Diese Tablette weist herstellungsbedingt 0,001 bis 5 g, vorzugsweise 0, 2 g bis 1 g, bezogen auf 100 g des Mittels, wenigstens eines Gleitmittels in Form eines (Matrizen)formtrennmittels auf. Beispielhaft seien hier siliconisiertes Talcum, Cetyl-Talcum, Magnesiumstearat, PEG 4000-6000, Stearinsäure, Cetylalkohol, Paraffin, Bienenwachs, hydrierte Fette und Öle und sonstige physiologisch verträgliche Formtrennmittel eingesetzt. Eine Übersicht hierüber gibt die Monographie von Rudolf Voigt im Kapitel Tabletten. Hier ist es besonders bevorzugt, als Tablette eine Oblongtablette einzusetzen.

In einer weiteren bevorzugten Ausführungsform weist die Tablette einen löslichen Überzug auf, der die Tablette überzieht. Verwiesen wird hier beispielsweise auf die Monographie von Schöffling-Krause auf Seite 144 - 148. Dieser Überzug erfolgt üblicherweise in Mengen von 0,1 g bis 50 g, vorzugsweise 1 g bis 20 g, bezogen auf 100 g des Mittels, und kann beispielsweise aus filmbildenden, durch magensäurelöslichen Lacken beispielsweise einem Andecksirup auf Basis von Hydrogelen oder Andeckpulvern, Farbpigmentsuspensionen, einem Glättesirup oder einer Hartwachslösung oder -suspension bestehen. Weiterhin verwendet werden Filmüberzüge mit speichelresistenten, magensaftlöslichen Polymeren, beispielsweise Polyacrylaten. Weitere Filmüberzüge sind lösliche Cellulosederivate wie Hydroxypropylcellulose. Eine Übersicht über geeignete Filmbildner gibt wiederum die Monographie von Voigt im Kapitel Dragees ab Seite 261 ff. Weiterhin geeignet sind Überzüge nach dem Verfahren der Zuckerdragierung, wie ebenfalls aus dem Kapitel "Dragees" in der Monographie von Voigt ersichtlich.

Nach einer weiteren bevorzugten Ausführungsform ist der Collagenschwamm verkapselt, liegt also als eine in Magensaft lösliche Kapsel vor, beispielsweise in Form einer Weichgelatinekapsel, einer Gelatinesteckkapsel oder als Kapsel mit einer modifizierten Wirkstofffreigabe. In diesem Zusammenhang verweisen wir beispielhaft auf die Monographie Arzneimittelformenlehre von Frau Schöffling-Krause, Stuttgart 1987, S. 118 - 130 und die dort beschrieben Herstellmethoden und Maschinen sowie auf das Kapitel Kapseln im Buch von Rudolf Voigt, Pharmazeutische Technologie für Studium und Beruf, Verlag Ullstein Mosby, Berlin 1993 und die dort beschriebenen Herstellmethoden und Maschinen hin.

Nach einer weiteren bevorzugten Ausführungsform enthält der Collagenschwamm wenigstens einen Wirkstoff und/oder Zusatzstoff. Die Wirkstoffe werden zu verschiedenen Zeitpunkten bei der Herstellung dem Collagenschwamm zugesetzt. Zusatzstoffe sind beispielsweise zugelassene Farbstoffe wie Carotinoide oder Vitamine wie z.B. Vitamin B 2. Wirkstoffe wie z.B. Omeprazol können ebenso zu verschiedenen Zeitpunkten, z.B. vor dem Komprimieren den Schwämmen, zugesetzt werden.

Nach einer weiteren bevorzugten Ausführungsform des erfindungsgemäß verwendeten Mittels ist der Wirkstoff in einer Matrix, Umhüllung, Einbettung und/oder einem anderen die Freisetzung steuernden Trägermaterial enthalten. Hierdurch kommt es zu einer Wirkstofffreisetzung durch Membrandiffusion, Porendiffusion, Quellung, Erosion, Porendiffusion aus der Matrix, zu einer Quellung mit Diffusion sowie zu einer Quellung mit Zerfall. Verwiesen wird hier beispielsweise auf die Monographie von R. Voigt, Kapitel Perorale Depotarzneimittelformen". Insbesondere wird im vorliegenden Falle als die Freisetzung steuerndes Trägermaterial Hydroxypropylmethylcellulose verwendet.

Der vorliegenden Erfindung liegt weiterhin die Aufgabe zugrunde, ein Verfahren zu Herstellung des vorgenannten Mittels bereitzustellen.

Die Erfindung betrifft somit weiterhin ein Verfahren zur Herstellung des vorgenannten Mittels, welches dadurch gekennzeichnet ist, dass man einen feinporigen Schwamm aus Collagen mit einer Dichte von 0,005 g/cm³ bis 1,0 g/cm³, der ggf. vor dem Pressvorgang mit wenigstens einem Wirkstoff und/oder Zusatzstoff behandelt worden ist sowie ggf. unter Verwendung eines Formentrennmittels auf die Hälfte bis ein Fünfzigstel, vorzugsweise ein Drittel bis ein Dreißigstel seiner Ursprungsgröße verpresst wird und ggf. mit einer in Magensaft löslichen Kapsel umgibt.

Nach einer weiteren bevorzugten Ausführungsform des erfindungsgemäßen Verfahrens erfolgt die Kombination des feinporigen Schwamms mit einer Trägerschicht für mindestens einen Wirkstoff. Die Trägerschicht wird entsprechend dem Herstellungsverfahren von Schichtentabletten auf den vorkomprimierten Schwamm aufgepresst.

Nach einer bevorzugten Ausführungsform des erfindungsgemäßen Verfahrens erfolgt die Behandlung des feinporigen Schwamms mit wenigstens einem Wirkstoff und/oder Zusatzstoff vor oder während einem wenigstens einstufigen Pressvorgang. Dies geschieht bevorzugt in der Weise, dass man die Wirk- und / oder Zusatzstoffe in an sich bekannter Weise auf den Träger in Form des Schwamms aufbringt, beispielsweise entweder pur, in einem Lösemittel gelöst oder als Dispersion in Form einer Emulsion oder Suspension.

Die Herstellung und Verpressung der Schwämme erfolgt beispielsweise nach Vorverdichtung des Schwamms nach Einlegen in eine Exenterpresse mit einem für die Tablettenherstellung üblichen Presswerkzeug mit Unter- und Oberstempel und geeigneter Matrize (z.B. Oblongform, 1,8 x 0,9 cm). Unter Ausstanzen erfolgt die Verpressung eines vorkomprimierten Schwammes zu einer Tablette mit einer Dicke von 4 mm. Wirkstoffe können auch vor dem Gefriertrocknungsprozess in die Collagendispersion eingearbeitet werden.

Als Wirkstoff im Sinne der vorliegenden Erfindung kann sowohl ein biologisch aktiver Stoff wie beispielsweise ein Kosmetikum oder ein Pharmazeutikum eingesetzt werden, welches insbesondere während der Verweilzeit im Magen freigesetzt werden kann.

Als kosmetisch wirksame Stoffe können hierbei beispielsweise Vitamine, wie β-Carotin oder Fettsäuren, die über eine systemische Einwirkung eine kosmetisch vorteilhafte Wirkung auf die Haut im Sinne einer Hautregeneration oder einer Hautbräunung bewirken können.

Darüber hinaus können als derartige Wirkstoffe auch Mineralien und Spurenelemente eingesetzt werden.

Gegenstand der vorliegenden Erfindung ist die Verwendung eines komprimierten, nicht-toxischen Collagenschwamms als Mittel zur Appetithemmung.

Dies geschieht bevorzugt in der Weise, dass man zusätzlich in diesen Collagenschwamm noch nahrungsergänzende Stoffe, insbesondere Vitamine, Mineralien, Fettsäuren und/oder Ballaststoffe zufügt oder einarbeitet.

Als derartige Nahrungsergänzungsstoffe sind zunächst Vitamine, die sich bekanntlich einerseits in fettlösliche Vitamine wie beispielsweise Retinol, Retinsäure, Retinal, Calciferole, d.h. die D-Vitamine, die Tocopherole oder E-Vitamine und die K-Vitamine oder Phyllochinone aufteilen. Ein Mangel an A-Vitaminen bewirkt Nachtblindheit, ein Mangel an D-Vitaminen bewirkt Rachitis und ein Mangel an E-Vitaminen vermehrt die oxidative Hämolyseeignung, bewirkt hämolytische Anämien, Ödeme und eine verstärkte Erregbarkeit. Ein Mangel an K-Vitaminen bewirkt eine Störung der Blutgerinnung und der Hämorrhagien.

Eine weitere Gruppe, die erfindungsgemäß in den nahrungsergänzenden Mitteln eingesetzt werden kann sind wasserlösliche Vitamine, wie Vitamine der B-Gruppe, wie beispielsweise Vitamin B1, das Thiamin, das Riboflavin, das Pyridoxin, die Nikotinsäure, die Corrionoide, die Folsäure und als weitere Gruppe die Ascorbinsäure bzw. das Vitamin C. Bei einem Mangel an Thiamin kommt es zur Beri-Beri-Krankheit, bei einem Mangel an Riboflavin kann sich die Cornea des Auges entzündlich verändern und es erfolgt eine erhöhte Vaskularisation. Bei einem Mangel an B6-Vitaminen kann es zu einer seborrhoischen Dermatitis, hypochromen Anämie, peripheren Neuritiden sowie zerebralen Konvulsionen kommen. Auch in der Schwangerschaft und nach einer Strahlentherapie ist ein erhöhter Bedarf an Vitamin B6 gegeben. Bei einem Mangel an Nikotinsäure kommt es zur Pellagra-Krankheit, bei einer Unterversorgung an Corrinoiden kommt es zu einer perniziösen Anämie oder selbst zu einer funikulären Myelose. Bei einer Unterversorgung an Folsäure kommt es zu Problemen bei der Schwangerschaft. Bei einer Unterversorgung an Ascorbinsäure kommt es zum Skorbut und zu der Möller-Barlowschen Erkrankung.

Die Tageszufuhr an Vitaminen durch die erfindungsgemäßen Mittel ergibt sich beispielsweise aus der Empfehlung für die Nährstoffzufuhr, wie sie von der DGE zusammengestellt worden ist. Typische Tageszufuhren an Vitaminen sind beispielsweise weiterhin in der Monographie von Forth Pharmakologie und Toxikologie" 4. Auflage, 1983, Seite 401 genannt.

Weitere Nahrungs-ergänzungsmittel sind Mineralien oder Spurenelemente, welche prophylaktisch oder therapeutisch zugeführt werden sollten. Dies sind beispielsweise Eisen, Zink, Kupfer, Mangan, Molybdän, Jod, Kobalt und Seien als essentielle Elemente für den menschlichen Körper. Bezüglich eines typischen Tagesbedarfes verweisen wir auf die vorgenannte Monographie von Forth, hier auf die Tabelle auf Seite 416.

Neben den essentiellen Elementen für den menschlichen Körper ist es in vielen Fällen auch erforderlich Calcium zuzuführen, welches nicht nur für den Knochen und Zellaufbau notwendig ist, sondern für den ganzen Körpermetabolismus. Das üblicherweise durch Nahrungsmittel vom Körper aufgenommene Calcium genügt nicht in allen Fällen den vorliegenden Erfordernissen. Calcium verleiht Knochen und Zähnen ihre Festigkeit.

Weiteres wesentliches Element, was erfindungsgemäß zugeführt werden kann, ist Kalium, welches eine aktive Rolle bei der Regulation des osmotischen Drucks innerhalb der Zelle spielt. Kalium ist eine Komponente des Verdauungstraktes des Magens und Darms und wird schnell resorbiert.

Weitere wesentliche für eine Nahrungsergänzung erforderliche Komponente ist das Magnesium, welches die Muskelfunktion beeinflußt. Magnesium ist ein wesentliches Nahrungsmittel, welches in nahezu allen Zellen auftaucht und die Aktivierung von Enzymen in Bezug auf den Energiemetabolismus steuert.

Das erfindungsgemäß verwendete Mittel weist beispielsweise pro Einheit einen Gehalt des tierischen Proteins Collagen von etwa 0,22 g auf, entsprechend einem mittleren physiologischen Verbrennungswert von etwa 4,0 kJ, entsprechend etwa 1,0 kcal, entsprechend 18 kJ oder etwa 4,4 kcal pro g des erfindungsgemäßen Mittels.

Die Einnahmeempfehlung des erfindungsgemäßen Mittels pro Tag beträgt im allgemeinen bis zu 10 Einheiten, vorzugsweise 6 bis 9 Einheiten, 3 mal am Tag, 1/2 Stunden vor der Mahlzeit.

Die vorliegende Erfindung wird nachfolgend durch Herstellungs- und Anwendungsbeispiele erläutert und mit dem Stand der Technik verglichen. Hierin werden Teile stets als Gewichtsteile angegeben.

### Herstellungsbeispiel 1 (peroral zu verabreichendes Mittel ohne Wirksubstanz):

Ein Collagenschwamm (Länge 46 cm, Breite 8 cm, Dicke 1,3 cm) mit einem Gewicht von 12,8 g wird mit Hilfe einer pneumatischen Presse auf eine Breite von 1,5 cm vorkomprimiert, so dass sich ein Streifen (Länge 46 cm, Breite 1,5 cm, Dicke 1,3 cm) ergibt.

Das Ausgangsmaterial weist folgende Kenndaten auf:
- eine Wasseraufnahmefähigkeit > 3.000 %,
- eine Trockenmasse von 89% +/- 5%,
- einen Aschegehalt < 3 %,
- einen pH-Wert von 3,2 +/- 0,2 sowie
- eine Dichte von 30 mg/cm +/- 10%.

Der Streifen wird abschnittsweise in eine Exzenterpresse (Tablettenpresse EK 0, Fa. Korsch, Berlin) eingebracht und unter Ainsstanzen des Materials mit Hilfe eines Unter- und Oberstempels und einer Matrize zu einer Oblongtablette (19 mm x 8 mm) mit jeweils vier Kerben auf Ober- und Unterseite verpresst. Die Tabletten weisen eine Dicke von 4 mm bei einem Gewicht von etwa 400 mg auf. Die Tabletten sind nach dem Verpressen formstabil. Die Presslinge expandieren in Wasser von 37 °C unter Aufnahme von Flüssigkeit innerhalb von maximal 5 Minuten zu einem Schwamm (1,9 cm x 0,8 cm x 8 cm).

Das so erhaltene peroral verabreichbare Mittel zeigte auch nach einer Lagerung von wenigstens 2 Monaten unter Luftfeuchtigkeit keine Volumenvergrösserung.

### Herstellungsbeispiel 2 (peroral verabreichbares Mittel mit Formentrennmittel)

Wie in Herstellungsbeispiel 1 wird ein Collagenschwamm zu einem Streifen vorkomprimiert. Die Ober- und Unterseite des Streitens wird vor dem Komprimieren mit dem pulverförmigen Formentrennmittel Magnesiumstearat beschichtet. Im vorliegenden Beispiel wurden pro Streifen 65 mg Magnesiumstearat verwendet. Die Tabletten enthalten jeweils etwa 2 mg Formentrennmittel auf der Oberfläche. Durch das hydrophobe Formentrennmittel wird die initiale Expansion des Schwammes innerhalb der ersten Minute verzögert. In Wasser von 37 °C expandieren die Presslinge unter Aufnahme von Flüssigkeit innerhalb von 5 Minuten zu einem Schwamm (1,9 x 0,8 x 8 cm).

Das so erhaltene peroral verabreichbare Mittel zeigte auch nach 2 Monaten unter Luftfeuchtigkeit keine Volumenvergrösserung.

### Herstellungsbeispiel 3 (peroral verabreichbares Mittel als Appetithemmer)

Die Herstellung erfolgte entsprechend Herstellungsbeispiel 1. Dem Schwamm werden 5 g Myristinsäure, Natriummyristat, Ammoniummyristat, Triethanolaminmyristat oder andere Derivate von Fettsäuren, bezogen auf 100 g des Mittels, zugesetzt. Das so erhaltene peroral verabreichbare Mittel kann als Appetithemmer eingesetzt werden.

Das so erhaltene peroral verabreichbare Mittel kann als Appetithemmer eingesetzt werden und zeigt auch nach einer mindestens 2-monatigen Lagerung bei Luftfeuchtigkeit keine Volumenvergrösserung.

### Herstellungsbeispiel 4 (peroral verabreichbares Nahrungsergänzungsmittel)

Herstellungsbeispiel 1 wurde wiederholt, wobei zusätzlich 5 g natürliches Vitamin E bezogen auf 100 g des Mittels, hinzugefügt worden sind.

Das so erhaltene Nahrungsergänzungsmittel zeigte auch nach einer 2-monatigen Lagerung bei Luftfeuchtigkeit keine Volumenvergrösserung.

### Anwendungsbeispiel 1 (in vitro)

In künstlichem Magensaft nach dem US-Arzneibuch (USP XXIII) mit Pepsinzusatz wurde der in Herstellungsbeispiel 1 beschriebene Pressling bei 37 °C auf seine Abbaubarkeit innerhalb des Verdauungstraktes untersucht. Hierbei ergab sich nach 240 Minuten ein beginnender Abbau des Collagenschwamms. Nach Wechsel des Magensaftes gegen künstlichen Darmsaft des US-Arzneibuchs (USP XXIII) mit Pankreatin zerfielen die Collagenschwämme nach 5,5 Stunden vollständig. Auch bei einer Einbringung in künstlichen Darmsaft erfolgte die vollständige Auflösung des Collagens innerhalb von 6 - 8 Stunden.

### Anwendungsbeispiel 2 (in vitro)

In 0,01 N Salzsäurelösung bei 37 °C wurde die Freisetzung von Riboflavin (Vitamin B2) aus einem komprimierten Collagenschwamm gemäss Herstellungsbeispiel 4 untersucht. Die Freisetzung von Riboflavin erfolgt über einen Zeitraum von mehr als 18 Stunden. Nach 1 Stunde sind 3 mg, nach 2 Stunden sind 5,5 mg, nach 3 Stunden sind 7 mg, nach 4 Stunden sind 8,2 mg, nach 5 Stunden sind 9,2 mg, nach 10 Stunden sind 12,9 mg und nach 15 Stunden sind 16,4 mg Riboflavin freigesetzt.

### Anwendungsbeispiel 3 (in vivo Studie der Magenverweilzeit)

2 Collagenschwämme gemäss Herstellungsbeispiel 4, die mit 10 g Riboflavin, bezogen auf 100 g des Collagenschwamms, beladen waren, wurden von je 12 Probanden nach Fasten über Nacht vor der Mahlzeit oder anstelle einer Mahlzeit mit 200 ml Leitungswasser eingenommen. Standardisierte Mahlzeiten wurden 30 min nach Einnahme sowie nach 4 oder 8 Stunden gegeben (Zusammensetzung: 1 Weizenbrötchen, 10 g Butter, 40 g Käse). Stündlich nahmen die Probanden 200 g Wasser zu sich, um ausreichende Hammengen für die analytische Bestimmung von Riboflavin sicherzustellen.

Zur Untersuchung der Magenverweilzeit des Collagenschwamms wurde zur Untersuchung pharmazeutischer Zubereitungen etablierte nicht invasive Untersuchungsmethode angewendet, bei der indirekt über die Zeitdauer der renalen Ausscheidung von Riboflavin die Zeitdauer seines Verbleibs im Magen ermittelt wird. Riboflavin wird lediglich aus dem oberen Bereich des Dünndarms vom Körper aufgenommen. Bei einer Freisetzung von Riboflavin im Magen gelangt die aufgelöste Substanz in den Dickdarm, aus welchem die Aufnahme über einen aktiven Absorptionsmechanismus erfolgt. Die Zeitdauer der renalen Ausscheidung von Riboflavin wird mit der Magenverweilzeit des oral verabreichbaren Mittels korreliert. Es wurde gefunden, dass die renale Elimination von Riboflavin d.h. von mehr als 0,2 mg/h über einen mittleren Zeitraum von 9,9 Stunden (arithmetisches Mittel) erfolgt.

### Vergleichsbeispiel 1 und Anwendung hierzu (in vivo Studie der Magenverweilzeit bei Depottablette)

Anstelle eines Collagenschwamms gemäss vorbeschriebenem Ausführungsherstellungsbeispiel 4 wurde eine nicht expandierende Riboflavin-Depottablette (Mikrotablette) mit einem Durchmesser von 4 mm untersucht. Es wurde den Probanden jeweils 2 Tabletten pro Tag verabreicht. Es wurde gefunden, dass die renale Elimination von Riboflavin d.h. von mehr als 0,2 mg/h lediglich über einen Zeitraum von 5,6 Stunden (arithmetisches Mittel) anhält, also 4,3 Stunden kürzer ist.

## Patentansprüche

1. Verwendung eines komprimierten Collagen-Schwammes zur oralen Verabreichung zur Appetithemmung.

2. Verwendung nach Anspruch 1, **dadurch gekennzeichnet, dass** das Collagen aus dem Stamm Porifera stammt.

3. Verwendung nach Anspruch 1, **dadurch gekennzeichnet, dass** das Collagen aus Stütz- und Bindegeweben, Haut, Knochen und Knorpeln gewonnen ist.

4. Verwendung nach einem der vorstehenden Ansprüche, **dadurch gekennzeichnet, dass** der Collagen-Schwamm vor der Komprimierung eine Dichte von 0,005 bis 1 g/cm³ aufweist.

5. Verwendung nach einem der vorstehenden Ansprüche, **dadurch gekennzeichnet, dass** der Collagen-Schwamm weiterhin wenigstens einen Wirkstoff und / oder Zusatzstoff enthält.

6. Verwendung nach Anspruch 5, worin der Zusatzstoff ein Farbstoff ist.

7. Verwendung nach einem der vorstehenden Ansprüche, worin der komprimierte Kollagenschwamm erhältlich ist durch ein Verfahren, bei dem man einen feinporigen Schwamm aus Kollagen mit einer Dichte von 0,005 bis 1 g/cm³, der gegebenenfalls vor dem Pressvorgang mit wenigstens einem Wirkstoff und/oder Zusatzstoff behandelt worden ist, gegebenenfalls in Gegenwart eines Formentrennmittels auf die Hälfte bis ein Fünfzigstel, vorzugsweise ein Drittel bis Dreißigstel seiner Ursprungsgröße verpresst.

8. Verwendung nach Anspruch 7, **dadurch gekennzeichnet, dass** der Pressvorgang wenigstens einstufig durchgeführt wird.

## Claims

1. Use of a compressed collagen sponge for oral administration to suppress appetite.

2. Use according to Claim 1, **characterized in that** the collagen originates from the phylum Porifera.

3. Use according to Claim 1, **characterized in that** the collagen is obtained from supporting and connective tissue, skin, bone and cartilage.

4. Use according to one of the preceding claims, **characterized in that** the collagen sponge has a density of 0.005 to 1 g/cm³ before compression.

5. Use according to one of the preceding claims, **characterized in that** the collagen sponge also contains at least one active ingredient and/or additive.

6. Use according to Claim 5 wherein the additive is a colourant.

7. Use according to one of the preceding claims wherein the compressed collagen sponge can be obtained by a process in which a fine-pore collagen sponge with a density of 0.005 to 1 g/cm³, which has optionally been treated with at least one active ingredient and/or additive before the compression operation, is compressed to one half to one fiftieth, preferably one third to one thirtieth, of its original size, optionally in the presence of a mould release agent.

8. Use according to Claim 7, **characterized in that** the compression operation is carried out in at least one step.

## Revendications

1. Utilisation d'une éponge de collagène comprimé pour l'administration par voie buccale pour couper l'appétit.

2. Utilisation selon la revendication 1, **caractérisée en ce que** le collagène est issu de la souche Porifera.

3. Utilisation selon la revendication 1, **caractérisée en ce que** le collagène est extrait de tissus de soutien et de tissus conjonctifs, de peau, d'os et de cartilages.

4. Utilisation selon l'une quelconque des revendications précédentes, **caractérisée en ce qu'**avant sa compression, l'éponge de collagène présente une densité comprise entre 0,005 et 1 g /cm³.

5. Utilisation selon l'une quelconque des revendications précédentes, **caractérisée en ce que** l'éponge de collagène contient par ailleurs un principe actif et/ou un additif.

6. Utilisation selon la revendication 5, l'additif étant un colorant.

7. Utilisation selon l'une quelconque des revendications précédentes, l'éponge de collagène comprimé pouvant être obtenue par un procédé dans lequel on comprime le cas échéant en présence d'un agent de démoulage à la moitié et jusqu'au cinquantième, de préférence à un tiers et jusqu'au trentième de sa dimension initiale, une éponge de collagène à pores fins, d'une densité comprise entre 0,005 et 1 g / cm³ ayant été traitée le cas échéant avant le processus de compression à l'aide d'au moins un principe actif et/ou d'un additif.

8. Utilisation selon la revendication 7, **caractérisée en ce que** le processus de compression est au moins réalisé en une étape.
